# FASCICULE DE BREVET EUROPEEN

(11) **EP 3 638 156 B1**
(45) Date de publication et mention de la délivrance du brevet: **17.08.2022**
(21) Numéro de dépôt: 18742799.2
(22) Date de dépôt: 19.07.2018
(51) Int. Cl.: A61B 17/17, A61B 17/70, A61F 2/46, A61F 2/44

(54) **DISPOSITIF D'IMPLANT POUR LA RÉALISATION D'UNE ARTHRODÈSE RACHIDIENNE PAR VOIE POSTÉRIEURE AU NIVEAU D'UNE ARTICULATION FACETTAIRE**
IMPLANTATVORRICHTUNG ZUR DURCHFÜHRUNG EINER POSTERIOREN WIRBELSÄULENARTHRODESE AN EINEM FACETTENGELENK
IMPLANT DEVICE FOR PERFORMING POSTERIOR SPINAL ARTHRODESIS AT A FACET JOINT

(43) Date de publication de la demande: 22.04.2020
(73) Titulaire: SC Medica, 67000 Strasbourg (FR)
(72) Inventeur: SROUR, Camille, 67000 Strasbourg (FR); SROUR, Robin, 67200 Strasbourg (FR)
(74) Mandataire: Cabinet Bleger-Rhein-Poupon
(86) Numéro de dépôt international: PCT/EP2018/069690
(87) Numéro de publication internationale: WO 2019/016341

(56) Documents cités:
- WO-A1-02/03895
- FR-A1- 2 946 245
- US-A1- 2011 230 965

## Description

Dispositif d'implant pour la réalisation d'une arthrodèse rachidienne par voie postérieure au niveau d'une articulation facettaire.

La présente a pour objet un dispositif d'implant pour la réalisation d'une arthrodèse rachidienne par voie postérieure au niveau d'une articulation facettaire.

Une telle intervention, réalisée par voie postérieure, permet d'immobiliser deux vertèbres en faisant fusionner chacune des apophyses articulaires inférieures d'une vertèbre avec l'apophyse articulaire supérieure en regard, de la vertèbre inférieure voisine.

Il existe différentes techniques de réalisation d'une arthrodèse rachidienne. Par la voie postérieure, la plus aisée, on peut utiliser une plaque ou une barre(s) d'ostéosynthèse fixée au moyen de vis pédiculaires, ou bien des cages intersomatiques du type de celles décrites par exemple dans les documents US 2011/230965, FR 2 946 245 et WO 02/03895, destinées à prendre place dans l'espace intervertébral après enlèvement du disque vertébral.

L'arthrodèse rachidienne par voie postérieure au niveau d'une articulation facettaire, présente des avantages par rapport aux autres techniques fréquemment mises en œuvre. Cette technique est notamment moins traumatique et présente moins de risques.

Cette technique nécessite toutefois d'utiliser des moyens différents de ceux des autres techniques opératoires, ainsi les caractéristiques des cages intersomatiques, telles que celles de documents précités, ne sont pas adaptées à une utilisation pour ce mode opératoire.

On connaît déjà des dispositifs d'implant destinés à être insérés dans une articulation facettaire, et notamment celui décrit dans le document WO 2012154653, qui consiste en un élément en forme de coin, dont les deux faces opposées, destinées chacune à venir au contact d'une facette, présentent une surface munie de reliefs pour en assurer l'ancrage, et qui est percé d'une multiplicité de trous destinés au développement de la matière osseuse après dépôt d'un greffon osseux, tandis que la fixation est réalisée au moyen de deux vis, chacune vissée dans une apophyse. Ce dispositif d'implant reste complexe, ou plus particulièrement c'est sa mise en oeuvre qui est complexe.

La présente invention a pour but de proposer un dispositif d'implant pour la réalisation d'une arthrodèse rachidienne par voie postérieure au niveau d'une articulation facettaire, qui est de conception plus simple que ceux connus, et qui est également de mise en œuvre plus aisée.

Le dispositif d'implant pour la réalisation d'une arthrodèse rachidienne par voie postérieure au niveau d'une articulation facettaire selon l'invention, se caractérise en ce qu'il se présente sous la forme d'un élément plat, d'épaisseur constante, comprenant, pour la réception d'un greffon osseux, une large ouverture centrale lui conférant une forme sensiblement annulaire, et destiné à être introduit dans une cavité préalablement réalisée à l'emplacement de l'articulation facettaire à immobiliser ; en ce que ledit élément présente une forme de D, le côté arrondi constituant le bord distal d'introduction dans ladite cavité, tandis que le bord opposé, proximal rectiligne, comporte un moyen de solidarisation à un outil de maintien et de mise en place ; et en ce que les faces opposées dudit élément plat, comportent des reliefs qui consistent en des crans disposés perpendiculairement au sens d'introduction, chacun des crans présentant un profil de dent à deux pans dont l'un, celui orienté du côté dudit bord proximal est perpendiculaire ou sensiblement perpendiculaire au plan principal dudit élément, tandis que l'autre pan est incliné.

Selon une caractéristique additionnelle du dispositif d'implant selon l'invention, l'élément plat présente, du côté de son bord distal arrondi, une épaisseur allant en diminuant, en sorte de lui conférer à ce bord distal un profil en coin.

Selon une autre caractéristique additionnelle du dispositif selon l'invention, le moyen de solidarisation à un outil de maintien et de mise en place, consiste en un trou taraudé, d'axe parallèle au sens d'introduction.

Les avantages et les caractéristiques du dispositif d'implant selon l'invention, ressortiront plus clairement de la description qui suit et qui se rapporte au dessin annexé, lequel en représente un mode de réalisation non limitatif.

Dans le dessin annexé :
- la figure 1 représente une vue en plan du dispositif d'implant selon l'invention.
- la figure 2 représente une vue en coupe selon l'axe AA' de la figure 1.
- la figure 3 représente une vue du même implant en position après implantation, du côté droit entre des vertèbres L5 S1, et selon la direction de l'introduction.
- la figure 4 représente le même implant dans la même position, mais vue en transparence et selon un plan perpendiculaire.

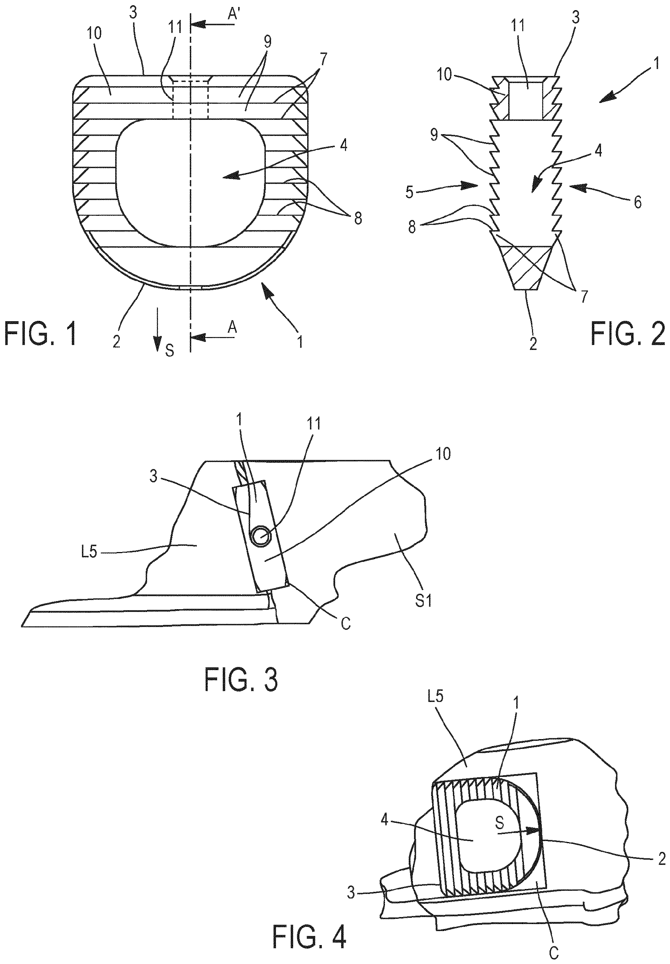

En référence aux figures 1 et 2, on peut voir un dispositif d'implant 1 pour la réalisation d'une arthrodèse au niveau d'une articulation facettaire selon l'invention.

On notera préalablement que le dispositif d'implant 1 selon l'invention est réalisé, non limitativement, en titane.

Ce dispositif d'implant 1 est sensiblement plat, comme cela est visible plus précisément sur la figure 2, et affecte la forme d'un D. Il comporte ainsi un côté distal 2 de forme arrondie et un côté proximal 3 rectiligne, tandis qu'est libéré centralement un large espace 4.

Chacune des deux faces 5 et 6 du dispositif d'implant 1, est garnie de crans 7 alignés transversalement par rapport à l'axe AA', destinés à la réalisation d'un ancrage dans la matière osseuse. Comme on peut le voir sur la figure 2, chacun des crans 7 comprend deux pans, un pan 8 disposé du côté proximal 3 rectiligne et qui est perpendiculaire ou sensiblement perpendiculaire au plan principal du dispositif d'implant 1, et un pan 9 disposé du côté distal 2, et qui est incliné.

Sur cette même figure 2, on peut également voir que, du côté distal 2, les deux faces 5 et 6 vont en se rapprochant, en sorte de donner à ce côté distal 2 un profil en coin.

On peut également constater sur ces figures, que la partie 10 du dispositif d'implant 1, formant le côté proximal 3, est percée, selon l'axe AA', d'un trou 11, taraudé pour permettre son assujettissement à l'extrémité d'un outil de mise en place.

En référence maintenant aux figures 3 et 4, on peut voir un dispositif d'implant 1 selon l'invention mis en place à l'emplacement de l'articulation facettaire entre les vertèbres L5 et S1, dont le côté droit est représenté partiellement, sachant que le côté gauche, non représenté est équipé de la même manière.

Avant la mise en place, la matière osseuse des deux vertèbres est découpée, au niveau de l'articulation facettaire, en sorte de créer une cavité C, partagée entre les deux vertèbres, en l'occurrence L5 et S1, et s'étendant dans le plan de contact.

Puis le dispositif d'implant 1 est introduit dans la cavité C, après avoir déposé dans son espace 4 un greffon osseux.

L'introduction du dispositif d'implant 1 est réalisée selon le sens de la flèche S reportée sur la figure 4, c'est-à-dire parallèlement à l'axe AA' de la figure 1 et en engageant en premier le côté distal 2 en forme de coin.

Le dispositif d'implant 1 selon l'invention est d'une grande simplicité de réalisation et de mise en œuvre.

## Revendications

1. Dispositif d'implant (1) pour la réalisation d'une arthrodèse rachidienne par voie postérieure au niveau d'une articulation facettaire, **caractérisé en ce qu'**il se présente sous la forme d'un élément plat, d'épaisseur constante, comprenant, pour la réception d'un greffon osseux, une large ouverture centrale (4) lui conférant une forme sensiblement annulaire, et destiné à être introduit dans une cavité (C) préalablement réalisée à l'emplacement de l'articulation facettaire à immobiliser ; **en ce que** ledit élément présente une forme de D, et comporte un côté arrondi (2) constituant le bord distal d'introduction dans ladite cavité (C), tandis qu'un bord opposé (3, 10), proximal rectiligne, comporte un moyen (11) de solidarisation à un outil de maintien et de mise en place ; et **en ce que** les faces (5, 6) opposées dudit élément plat, comportent des reliefs (7) qui consistent en des crans disposés perpendiculairement au sens d'introduction (S), chacun des crans (7) présentant un profil de dent à deux pans dont l'un (8), celui orienté du côté dudit bord proximal (3) est perpendiculaire ou sensiblement perpendiculaire au plan principal dudit élément, tandis que l'autre pan (9) est incliné.

2. Dispositif d'implant (1) selon la revendication 1, **caractérisé en ce que** l'élément plat présente, du côté du bord distal arrondi (2), une épaisseur allant en diminuant, en sorte de conférer à ce bord distal (2) un profil en coin.

3. Dispositif d'implant (1) selon la revendication 1 ou la revendication 2, **caractérisé en ce que** le moyen de solidarisation à un outil de maintien et de mise en place, consiste en un trou taraudé (11), d'axe parallèle au sens d'introduction (S).

## Patentansprüche

1. Implantatvorrichtung (1) für die Ausführung einer Wirbelsäulenarthrodese auf posteriorem Weg an einem Facettengelenk, **dadurch gekennzeichnet, dass** sie die Form eines flachen Elements mit konstanter Dicke aufweist, einschließlich, für die Aufnahme eines Knochentransplantats, einer großen zentralen Öffnung (4), die ihr eine im Wesentlichen ringförmige Form verleiht, und dafür bestimmt ist, in einen Hohlraum (C) eingeführt zu werden, der zuvor an der Stelle des zu immobilisierenden Facettengelenks ausgeführt wurde; dadurch, dass das Element eine D-Form aufweist und eine abgerundete Seite (2) umfasst, die die distale Kante zum Einführen in den Hohlraum (C) ausbildet, wohingegen eine proximale gerade gegenüberliegende Kante (3, 10) ein Mittel (11) zum Befestigen an einem Halte- und Positionierwerkzeug umfasst; und dadurch, dass die gegenüberliegenden Flächen (5, 6) des flachen Elements Reliefs (7) umfassen, die aus Einkerbungen bestehen, die senkrecht zu einer Einführrichtung (S) angeordnet sind, wobei jede der Einkerbungen (7) an zwei Seitenflächen ein Zahnprofil aufweist, darunter eine (8), diejenige, die der Seite der proximalen Kante (3) zugewandt ist, die senkrecht oder im Wesentlichen senkrecht zu einer Hauptebene des Elements ist, wohingegen die andere Seitenfläche (9) geneigt ist.

2. Implantatvorrichtung (1) nach Anspruch 1, **dadurch gekennzeichnet, dass** das flache Element auf der Seite der abgerundeten distalen Kante (2) eine abnehmende Dicke aufweist, um dieser distalen Kante (2) ein keilförmiges Profil zu verleihen.

3. Implantatvorrichtung (1) nach Anspruch 1 oder 2, **dadurch gekennzeichnet, dass** das Mittel zum Befestigen an einem Halte- und Positionierungswerkzeug aus einer Gewindebohrung (11) mit einer zu der Einführrichtung (S) parallelen Achse besteht.

## Claims

1. Implant device (1) for performing spinal arthrodesis via the posterior at a facet joint, **characterized in that** it is in the shape of a flat element of constant thickness, comprising, in order to receive a bone graft, a large central opening (4) which gives it a substantially annular shape, and which device is intended to be inserted into a cavity (C) previously made at the location of the facet joint to be immobilized; **in that** said element is D-shaped and comprises a rounded side (2) which constitutes the distal edge for being inserted into said cavity (C), while an opposite straight proximal edge (3, 10) comprises a means (11) for securing to a holding and positioning tool; and **in that** the opposite surfaces (5, 6) of said flat element comprise reliefs (7) which consist of notches arranged perpendicularly to the insertion direction (S), each of the notches (7) having a tooth profile with two faces of which one (8), the face oriented on the side of said proximal edge (3), is perpendicular or substantially perpendicular to the main plane of said element, while the other face (9) is inclined.

2. Implant device (1) according to claim 1, **characterized in that** the flat element has, on the side of the rounded distal edge (2), a thickness that decreases so as to give this distal edge (2) a wedge-shaped profile.

3. Implant device (1) according to either claim 1 or claim 2, **characterized in that** the means for securing to a holding and positioning tool consists of a tapped hole (11) having an axis parallel to the insertion direction (S).
